# EUROPEAN PATENT SPECIFICATION

(11) **EP 0 925 846 B1**
(45) Date of publication and mention of the grant of the patent: **10.03.2004**
(21) Application number: 98124362.9
(22) Date of filing: 22.12.1998
(51) Int. Cl.: B05D 1/04, B05D 1/06, B05D 5/00

(54) **Process for producing coated functional articles**
Verfahren zur Herstellung von funktionellen Beschichtungen auf Gegenständen
Procédé pour produire des objets ayant un revêtement fonctionnel

(30) Priority: 26.12.1997 JP 36878497
(43) Date of publication of application: 30.06.1999
(73) Proprietor: Kabushiki Kaisha Daisyo, Osaka-shi, Osaka 540-0035 (JP)
(72) Inventor: Kinoshita, Yoshiko, c/o K.K. Daisyo, Osaka-shi, Osaka 540-0035 (JP)
(74) Representative: Gille Hrabal Struck Neidlein Prop Roos

(56) References cited:
- EP-A- 0 633 064
- WO-A-97/00134
- GB-A- 963 557
- US-A- 5 219 493

## Description

### FIELD OF THE INVENTION

The present invention relates to a commercially advantageous process for producing coated functional articles having useful antimicrobial and/or deodorant functions.

### PRIOR ART

Today, the concept of environmental improvement from antimicrobial and deodorization points of view is pervading everywhere, e.g. in homes, business establishments, schools, hospitals, public facilities, and transportation facilities.

Recently, inorganic powders having photocatalytic activity represented by ultrafine powders of titanium oxide have been attracting attention as antimicrobial agents or deodorizing agents. Thus, a variety of substrates can be provided with antimicrobial and deodorant properties by kneading such photocatalytic inorganic powders into resin molding compositions for the production of fiber, film, and other shaped articles, formulating the powders in the raw material batches for ceramic products prior to firing, or applying them to the substrate surfaces of metal, plastic, glass, porcelain, woven fabric, wood, etc. by coating or dipping. Since photocatalytic inorganic powders require light for the expression of its functional properties (antimicrobial and deodorizing properties), it is common practice to somehow associate such products with a light source such as a built-in lamp, e.g. a UV lamp, when the end products are intended to be used in places not well exposed to sunlight or illumination light.

The technology of formulating, adding as an internal additive, or otherwise incorporating photocatalytic inorganic powders in the course of manufacture of end products may be suitable for high-production items but is not necessarily suited to the manufacture of various products in small lots. Moreover, since the amounts of photocatalytic inorganic particles which would be exposed on the substrate surfaces are small in relation to their level of addition, the practice is costwise not advantageous and, moreover, tends to alter the physical properties of the products.

On the other hand, the technology of covering substrate articles with photocatalytic inorganic powders by the coating or dipping technique can be applied to those products which are already existing and, hence, has a wide range of applicability. This technology is advantageous from the cost viewpoint, too, since the necessary functional layer can be formed selectively on the surface. Another advantage is that the risk for alterations in the physical properties of end products is low.

However, this technology of covering substrate articles with photocatalytic inorganic powders by the coating or dipping technique is more or less effective in providing uniform coats on flat surfaces but this is not true with substrates having complicated surfaces and in order to insure uniform coats on such surfaces, special equipment, much labor, and high degrees of skill are required. Moreover, even if such requirements are fulfilled, uniform coating can still be hardly obtained and inadequacies are sometimes felt in the provision of required functions. Furthermore, the ordinary coating method comprising coating or dipping substrates is poor in deposition efficiency, hence the loss of expensive photocatalytic inorganic powders amounts to a high percentage. Further, the coat thickness tends to become excessive, so that the feel and other surface properties of the end product are sometimes altered.

In addition, when substrate articles are coated with photocatalytic inorganic powders by the ordinary technique, the necessary functions (antimicrobial and deodorant functions) may fail to attain the expected levels. This is probably because a binder has to be used in a sufficiently large amount to provide a thick coat insuring adequate adhesion to the substrate. Thus, a large proportion of the photocatalytic inorganic particles used are buried in the binder, with the result that the proportion of those photocatalytic inorganic particles which should contribute to expression of the required functions becomes small.

Under the circumstances, the object of the present invention is to provide a commercially advantageous production technology capable of providing a thin layer of photocatalytic inorganic particles with good uniformity and ease on a substrate surface and, despite the thin coating thickness, providing a functional coated article capable of discharging the necessary functions.

GB 963 557A discloses a process comprising powdery polyepoxide particles which can contain titanium oxide as a filler in an atomised form given by means of an electrostatic coater. The particles are preferably prepared by melting. WO 97 00134A and EP 0 923 988A describe application of a photocatalyst layer composed of a metal oxide gel on top of an adhesive layer by spray coating. US-A-5 219 493 refers to electrostatic deposition for applying pigments thoroughly embedded into a binder in order to yield a smooth surface.

### Summary of the invention

The present invention refers to a process for producing coated functional articles as defined in appended claim 1 and which comprises applying a liquid material comprising ultrafine titanium oxide-based photocatalytic inorganic particles having an X-ray diameter of not more than 100 nm, an inorganic binder or a binder convertible to an inorganic substance, and a vehicle, in an atomized form given by means of an electrostatic coater, directly to the surface of an article to be coated at least the surface of which is electroconductive or has been treated for rendering the same electroconductive beforehand, to thereby cause said material to be adsorbed on said surface.

In accordance with a preferred embodiment of the invention the process comprises suspending hangers from a rail so that they can move and suspending the articles to be coated from the respective hangers, then moving the hangers having the articles through an electric spraying zone and a drying zone in that order.

### DETAILED DESCRIPTION OF THE INVENTION

In the following, the present invention is described in further detail.

The substrate includes articles of any and all profiles, e.g. articles having planar surfaces, three-dimensionally curved surfaces, or convex and/or concave surfaces, even articles which must be coated on both the face and reverse sides, and articles having complicated geometries. This constitutes one of the outstanding characteristics of the invention.

As the material of the substrate article which is coatable, there may be mentioned almost all solid substances, such as metals, coated metals, plastics, ceramics, glass, natural organic or inorganic materials.

As examples of the substrate article, there may be mentioned phones, toilet seats, sanitary earthenware, door knobs and handles, jars, bottles, ornaments, straps, toothbrushes, microphones, handles of luggage and bags, pots for beverages, umbrella or parasol handles, illuminating lamps, stationery (writing materials etc.), daily necessities (wash-line poles etc.), toiletries, bathroom articles, kitchen utensils, household appliances, chairs, footwear, paper, woven fabrics, nonwoven fabrics, knit fabrics, operating buttons, office automation (OA) equipment, cabinets, interior decoration materials, building materials, medical instruments, dental instruments, sports equipment and sporting goods, which are intended for use in homes, industries, public institutions or communities, transportation facilities, hospitals, horticulture, agriculture, livestock raising, marine products industry, sporting, or education, for instance.

When at least the surface of the article to be coated is electroconductive, the article can be submitted directly to the coating step to be described later herein.

When the article to be coated is not electroconductive, it is necessary to subject this to pretreatment for rendering the same electroconductive. A method useful for this conductivity-imparting treatment typically comprises treating the substrate article with a conductivity-providing treatment solution or dispersion prepared by dissolving or finely dispersing an agent capable of rendering the surface in question conductive (conductivity-providing agent) in a vehicle, to thereby reduce the surface resistance of the substrate article to about 10⁹Ω·cm or 10⁸Ω·cm or below.

Particularly important as the conductivity-providing agent are antistatic grade surfactants . As such surfactants, there may, e.g., be mentioned cationic surfactants (e.g. quaternary amine salts), anionic surfactants (e.g. sulfonic acid salts, phosphoric acid salts) amphoteric surfactants (e.g. alkylbetaines, alkylimidazolines), nonionic surfactants (e.g. polyoxyethylenealkylamines, polyoxyethylene alkyl ethers, glycerol fatty acid esters). Also useful as said agent are ionic conductive polymers such as cationic conductive polymers (e.g. polyvinylbenzyltrimethylammonium salts) and anionic conductive polymers (e.g. polystyrenesulfonic acid salts). It is to be noted that these are given only for illustrative purposes. Thus, a great variety of agents known as antistatic agents or conductivity-providing agents can be used.

Useful as the vehicle are organic solvents and inorganic solvents (e.g. water), inclusive of mixed solvents. It is important to see that the vehicle will not affect the article to be coated and has an adequate degree of volatility. Typical examples of the vehicle are alcohols such as methanol, ethanol and isopropyl alcohol.

The concentration of said conductivity-providing agent in said pretreatment solution or dispersion is generally about 0.01 to 2% by weight (in particular, 0.1 to 1% by weight), for instance.

The treatment of the substrate article with the conductivity-providing liquid can be performed by the dipping or shower technique or, more practically and desirably, by the spray technique (in particular, the air spray technique) which hardly gives rise to pools.

Then, according to the present invention, the above-mentioned liquid material containing inorganic particles having photocatalytic activity, in an atomized form given by means of an electrostatic coater, is applied to the surface of the substrate article at least the surface of which is electroconductive or has been treated for rendering the same electroconductive beforehand, to thereby cause said material to be adsorbed on said surface.

The principle of electrostatic coating consists in using the substrate article, which is earthed, as a positive electrode and applying a high negative voltage to the tip of a coating gun, which serves as a negative electrode, to thereby create an electrostatic field between the electrodes, whereby the sprayed particles of the coating composition are negatively charged and deposited on the substrate article. By actual site observation, it can be seen by the eye that the particles sprayed are adsorbed onto the substrate article as if a fog were rapidly clearing away. Two modes of electrostatic coating are feasible. Thus, the coating composition is applied in the form of a liquid (droplets). Spraying in the form of a liquid (fine dispersion), namely electrostatic liquid coating, is advantageous since the fixation after adsorption on the substrate article is easy to effect and there is no limitation on the material of the substrate article.

The liquid material (fine dispersion) containing ultrafine titanium oxide-based inorganic particles having photocatalytic activity (in the following called photocatalytic inorganic particles or powder) comprises a binder, a vehicle (or a vehicle serving as a binder as well), and the photocatalytic inorganic particles dispersed in said binder and/or vehicle.

The ultrafine titanium oxide powders have an X ray diameter of 100 nm or below. Ultrafine titanium oxide powders surface-modified with a metal (e.g. gold, silver, copper, platinum, zinc, silicon, iron) or a metal compound (e.g. zinc oxide, silicon oxide) are also suited for use.

Suited for use as the binder is an inorganic binder or a binder convertible to an inorganic substance.

Suited for use as the above-mentioned inorganic binder are, e.g., alumina sol and silica sol.

Suited for use as the binder convertible to an inorganic substance are tetramethoxysilane, tetraethoxysilane, trimethylmethoxysilane, methyltrimethoxysilane, methyltriethoxysilane, other alkoxysilanes, or oligomers of these, as well as other metal alkoxides. The alkoxysilanes are readily hydrolyzed after application to the substrate surface to form silanol groups, which are further crosslinked, and finally converted to an inorganic substance, SiO₂.

As the vehicle, there may be mentioned water, water-miscible organic solvents, mixtures of water and a water-miscible solvent (s) , and other organic solvents. Typical examples of the water-miscible organic solvent are alcohols such as methanol, ethanol and isopropyl alcohol.

The concentration of solids in the fine dispersion is appropriately about 1 to 30% by weight, for instance, but is not limited to said range. The ratio of the photocatalytic inorganic powder to the binder is generally 20:80 to 95:5 by weight, but is not limited to said range. When the proportion of the binder is too small, the photocatalytic inorganic particles after application to the substrate article tend to fall away. When, conversely, the proportion of the photocatalytic inorganic powder is too small, its functions (deodorant and antimicrobial functions) will not be fully expressed. The range mentioned above for the solids concentration and the range mentioned above for the photocatalytic inorganic powder/binder ratio are no more than reference ranges. The optimum conditions should be selected based on experiments.

suited for use as the electrostatic coater is an electrostatic coater of the electric spraying type, such as a bell type electrostatic coater or a disk type electrostatic coater. Each type has a high deposition efficiency, hence is particularly suitable for the purposes of the present invention. In addition to the electric spraying type equipment, an electrostatic coater of the air spraying type or airless spraying type are also suited for use.

Following electrostatic coating, spontaneous drying or forced drying is effected. The coated functional article thus obtained has a structure comprising a uniform thin layer of photocatalytic inorganic particles formed on the surface of the substrate article either directly or through the intermediary of a layer formed by the conductivity-providing treatment.

The present invention, which employs the electrostatic coating technique, makes it possible for the liquid applied to arrive at the whole substrate surface, irrespective of its shape or form, to thereby readily form a uniform thin surface layer containing the photocatalytic inorganic particles.

The coated functional article produced by the process of the present invention produces good antimicrobial and deodorant effects. The photocatalytic inorganic particles, which by themselves show an oxidizing action, when irradiated with light during their use for antimicrobial and deodorant purposes or when irradiated with light after their use for antimicrobial and deodorant purposes, restore the antimicrobial and deodorant activities, hence can be used repeatedly. The source of light is arbitrary; for example it may be a fluorescent lamp, white light, sunlight or an ultraviolet lamp. Since the photocatalytic inorganic particles can decompose organic substances, they effectively decompose organic dirt (e.g. finger marks, nicotine or tar formed upon smoking, tar from sticks of incense, soot formed upon cooking) , if adhering to said article, with the result that there is no possibility of fungal growth on the dirt.

### EXAMPLES

The following examples further illustrate the present invention.

### Production of coated functional articles

### Example 1

Hangers were suspended from a rail fixed on the ceiling so that they could move through a conductivity-providing treatment zone, an electric spraying zone, and a drying zone, in that order.

Cylindrical illumination lamps, animal-shaped ceramic ornaments (hollow), U-shaped door handles, artificial flowers, train strap rings, clinical chart files, personal computer mouse pads, electronic calculator housings, and ballpoint pens were provided as articles to be coated. The lamps were made of glass, the ornaments of ceramics, and the remaining articles of plastics.

These substrate articles were suspended from the respective hangers and sprayed with a conductivity-providing treatment solution, which was a 0.3% by weight solution of a conductivity-providing agent, i.e. a quaternary ammonium type surfactant, in methanol, in the conductivity-providing treatment zone by the air spraying technique. After the treatment, the substrate articles immediately became dry by spontaneous drying. They were thus rendered superficially electroconductive.

Ishihara Sangyo's photocatalytic titanium oxide/coating agent "ST-K03" was prepared as the fine dispersion (slurry) mainly composed of photocatalytic inorganic particles, an inorganic binder or a binder convertible to an inorganic substance, and a vehicle for dispersing or dissolving these. "ST-K03" had a solids concentration of 10% by weight and a TiO₂/binder weight ratio of 50/50, with the solvent comprising ethanol and water; it had a pH value of 1.5 and a viscosity of 5 cps. The X-ray diameter of the TiO₂ component contained therein was estimated to be 7 nm.

After the above conductivity-providing treatment, the substrate articles were transferred to the electric spraying zone, where the above fine dispersion was sprayed onto said substrate articles by means of an electrostatic coater of the electric spraying type. The fine dispersion sprayed was almost instantaneously adsorbed on the surface of each substrate article. The deposition efficiency was 90 to 95%.

After passing the articles through the drying zone, a thin coat layer could be obtained as formed on the surface of each article.

### Comparative Example 1

The above fine dispersion "ST-K03" was sprayed onto the same substrate articles as used in Example 1 by the air spraying technique and then dried.

### Comparative Example 2

The above fine dispersion "ST-K03" was sprayed onto the same substrate articles as used in Example 1 by the airless spraying technique and then dried.

### Deposit testing

The coated articles obtained in Example 1 and Comparative Examples 1 and 2 were examined for deposition efficiency and irregular deposition (absence or presence of uncoated portions, uniformity of film thickness in coated area) of the fine dispersion. For the irregular deposition examination, a dye detectable under a fluorescent lamp was incorporated in the fine dispersion and, after the spraying procedure, the coated articles were evaluated under a black light for the presence or absence of fluorescence-free portions (i.e. uncoated portions) and for the uniformity of fluorescence intensity in the fluorescence-emitting area (i.e. uniformity of coat film thickness). The results thus obtained are shown in Table 1. The number of samples per group was 10 and the evaluation was made based on those showing average results. The following three levels were used for judgment: ○ (good); Δ (average); × (poor).

**Table 1**

| Substrate article | Deposition efficiency | Fluorescence-free portions | Fluorescence intensity uniformity |
|---|---|---|---|
| | | | |

| Example 1 | | | |
|---|---|---|---|
| Ceramic ornaments | ○ 90-95% | ○ None | ○ Uniform and pale fluorescence |
| Strap rings | ○ 90-95% | ○ None | ○ Uniform and pale fluorescence |
| Door handles | ○ 90-95% | ○ None | ○ Uniform and pale fluorescence |
| Artificial flowers | ○ 90-95% | ○ None | ○ Uniform and pale fluorescence |

| Comparative Example 1 | | | |
|---|---|---|---|
| Ceramic ornaments | × 30-60% | Δ Some | Δ Not uniformly |
| Strap rings | × 30-60% | Δ Some | Δ Not very uniform |
| Door handles | × 30-60% | Δ Some | Δ Not very uniform |
| Artificial flowers | × 30-60% | × Many | × Marked variation |

| Comparative Example 2 | | | |
|---|---|---|---|
| Ceramic ornaments | × 30-60% | Δ Some | Δ Not uniformly |
| Strap rings | × 30-60% | Δ Some | Δ Not very uniform |
| Door handles | × 30-60% | Δ Some | Δ Not very uniform |
| Artificial flowers | × 30-60% | × Many | × Marked variation |

### Deodorization test

The coated functional articles (ceramic ornaments) obtained in Example 1 and Comparative Examples 1 and 2 were each placed in a 5-liter glass vessel with a black light placed therein. The vessel was tightly closed with a cover. As the samples for Example 1, three out of four randomly selected ones were used. As the samples for Comparative Example 1 or 2, three selected out of four having no fluorescence-free portions (uncoated portions) and showing least variations in fluorescence intensity were used. The black light was used as the source of light in lieu of sunlight or interior illumination so that a constant quantity of light could be obtained for measuring the odor component concentrations accurately at timed intervals.

Malodorous gases (ammonia, acetaldehyde and trimethylamine) were injected into the glass vessel 'from the top and irradiation with the black light was started. Immediately after and 4 hours after gas injection, the head space gas was sampled and the malodorous gas concentrations were measured using detecting tubes. The results are shown in Table 2. In Table 2, each of the numerical values (in ppm) is the mean of the three samples.

**Table 2**

| | NH₃ | | CH₃CHO | | N (CH₂CH₃)₃ | |
|---|---|---|---|---|---|---|
| | Initial | 4hr | Initial | 4hr | Initial | 4hr |
| Example 1 | 53 | 29 | 35 | 15 | 122 | 72 |
| Compar. Ex. 1 | 53 | 35 | 34 | 21 | 121 | 80 |
| Compar. Ex. 2 | 54 | 34 | 33 | 20 | 122 | 82 |

It can be seen from Table 2 that in spite of the use of the same spraying liquid (fine dispersion) for coating, the coated articles of Example 1 show stable and higher deodorization efficiencies as compared with the coated articles of Comparative Example 1 or 2. This is presumably because the coat film formed in Example 1 is thin and uniform and hence provides for a greater efficiency of contact between the particulate photocatalytic inorganic substance and the atmosphere.

### Antimicrobial test

A sample piece having a surface area of about 1,800 mm² was prepared by breaking the remaining one of the samples prepared for the above deodorization test.

The above sample piece was placed in 40 ml of water containing 0.1% by weight of a commercial culture medium (containing microorganisms that had fallen spontaneously). After tight closure, the whole was allowed to stand under indoor natural light. After 96 hours, the microbial growth was evaluated by measuring the transmittance at 600 nm. The results obtained are shown in Table 3.

**Table 3**

| Sample | Transmittance (%) | State |
|---|---|---|
| Blank | 84.1 | Cloudy, foul odor |
| Example 1 | 94.0 | Clear, odorless |
| Compar. Ex. 1 | 91.9 | Clear, odorless |
| Compar. Ex. 2 | 92.2 | Clear, odorless |

From Table 3, it is evident that while the articles of Example 1 and Comparative Examples 1 and 2 all show good antimicrobial activity, the results obtained with the articles of Example 1 are more favorable as compared with the articles of Comparative Examples 1 and 2.

### EFFECTS OF THE INVENTION

The present invention, which employs the electrostatic coating technique, makes it possible for the liquid applied to arrive at the whole substrate surface, irrespective of its shape or form, to thereby readily form a uniform thin coat layer containing the photocatalytic inorganic particles. Furthermore, the loss of photocatalytic inorganic particles is very small and this is advantageous from the materials cost viewpoint. In addition, the process can be performed using relatively simple equipment. The process of the invention is thus very useful as a commercial process.

The coated functional articles produced by the process of the present invention produce excellent antimicrobial and deodorant effects. The process can be applied to substrate articles already existing as products to thereby making the surrounding environment pleasant. The physical characteristics and appearance of substrate articles are never impaired on the occasion of coating.

The coats are very thin and uniform in thickness and this means an advantage from the cost viewpoint. Moreover, the photocatalytic inorganic particles in coat films as deposited on the surfaces of substrate articles show a large exposed surface area and, as a result, the efficiency of contact between the photocatalytic inorganic particles and odor components or microorganisms in the atmosphere is improved as compared with those articles having a thick coat and the antimicrobial and deodorant activities are much improved as compared with the articles coated by the conventional spraying, dipping or flow coating technique.

Furthermore, the photocatalytic inorganic particles decompose organic substances, so that they effectively decompose organic dirt (e.g. finger marks, nicotine or tar formed upon smoking, tar from sticks of incense, soot formed upon cooking), if adhering to the article, with the result that there is no possibility of fungal growth on the dirt.

## Claims

1. A process for producing coated functional articles which comprises applying a liquid material comprising ultrafine titanium oxide-based photocatalytic inorganic particles having an X-ray diameter of not more than 100 nm, an inorganic binder or a binder convertible to an inorganic substance, and a vehicle, in an atomized form given by means of an electrostatic coater, directly to the surface of an article to be coated at least the surface of which is electroconductive or has been treated for rendering the same electroconductive beforehand, to thereby cause said material to be adsorbed on said surface.

2. A process according to claim 1 which comprises
suspending hangers from a rail so that they can move, and suspending articles to be coated from the respective hangers,
then moving the hangers having the articles through an electric spraying zone wherein said liquid material is applied to the surface of the articles and through a drying zone, in that order.

## Patentansprüche

1. Verfahren zur Herstellung von beschichteten funktionellen Gegenständen, umfassend den Auftrag eines flüssigen Materials, das photokatalytische anorganische Teilchen auf der Basis von ultrafeinem Titanoxid mit einem Röntgenstrahlen-Durchmesser von nicht mehr als 100 nm, in einem anorganischen Bindemittel oder einem Bindemittel, das in eine anorganische Substanz umwandelbar ist und einem Vehikel enthält, in atomisierter Form, erhalten durch eine elektrostatische Beschichtungsvorrichtung, direkt auf die Oberfläche eines zu beschichtenden Gegenstandes wovon mindestens die Oberfläche elektrisch leitfähig ist oder vorher behandelt wurde, um sie elektrisch leitfähig zu machen, wodurch bewirkt wird, dass das Material an der Oberfläche adsorbiert wird.

2. Verfahren nach Anspruch 1, umfassend
das Aufhängen von Hängevorrichtungen an einer Schiene, so dass sie bewegt werden können und das Aufhängen der zu beschichtenden Gegenstände an die jeweiligen Hängevorrichtungen,
anschließendes Bewegen der Hängevorrichtungen mit den Gegenständen durch eine elektrische Sprühzone, in der das flüssige Material auf die Oberfläche der Gegenstände aufgetragen wird und durch eine Trocknungszone, in dieser Reihenfolge.

## Revendications

1. Procédé de production d'articles fonctionnels enduits qui comprend les étapes consistant à appliquer un matériau liquide comprenant des particules ultrafines inorganiques photocatalytiques à base d'oxyde de titane ayant un diamètre aux rayons X inférieur ou égal à 100 nm, un liant inorganique ou un liant convertible en une substance inorganique, et un véhicule, sous une forme atomisée conférée au moyen d'une machine à enduire électrostatique, directement sur la surface d'un article à enduire dont au moins la surface est électroconductrice ou a été préalablement traitée pour la rendre électroconductrice, pour causer ainsi l'absorption dudit matériau par ladite surface.

2. Procédé selon la revendication 1 comprenant les étapes consistant à :
suspendre des brides de suspension à partir d'un rail de manière à ce qu'elles puissent bouger, et suspendre des articles à enduire aux brides de suspension respectives ;
puis déplacer les brides de suspension ayant les articles suspendus à travers une zone de pulvérisation électrique dans laquelle ledit matériau liquide est appliqué à la surface des articles et à travers une zone de séchage.
